(19) Europäisches Patentamt / European Patent Office / Office européen des brevets

(11) EP 4 467 583 A1

(12) **EUROPEAN PATENT APPLICATION**
published in accordance with Art. 153(4) EPC

(43) Date of publication:
**27.11.2024 Bulletin 2024/48**

(21) Application number: **23877738.7**

(22) Date of filing: **13.10.2023**

(51) International Patent Classification (IPC):
***C08F 251/00*** (2006.01) ***C08F 220/34*** (2006.01)
***C08L 51/02*** (2006.01) ***C08L 101/00*** (2006.01)
***C08K 3/04*** (2006.01) ***C08K 3/34*** (2006.01)
***A61L 9/01*** (2006.01) ***C08B 31/00*** (2006.01)

(52) Cooperative Patent Classification (CPC):
**A61L 9/01; C08B 31/00; C08F 220/34; C08F 251/00; C08K 3/04; C08K 3/34; C08L 51/02; C08L 101/00**

(86) International application number:
**PCT/KR2023/015804**

(87) International publication number:
**WO 2024/080815 (18.04.2024 Gazette 2024/16)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **14.10.2022 KR 20220131942**
**12.10.2023 KR 20230136164**

(71) Applicant: **LG Chem, Ltd.**
**Yeongdeungpo-gu**
**Seoul 07336 (KR)**

(72) Inventors:
- **MOON, Hyeran**
**Daejeon 34122 (KR)**
- **KIM, Gi Hong**
**Daejeon 34122 (KR)**
- **LEE, Jeong Yun**
**Daejeon 34122 (KR)**
- **LEE, Hwanhee**
**Daejeon 34122 (KR)**
- **LEE, Donghwan**
**Daejeon 34122 (KR)**
- **CHOI, Hyungsam**
**Daejeon 34122 (KR)**
- **KWON, Seongjin**
**Daejeon 34122 (KR)**
- **YUN, Haesung**
**Daejeon 34122 (KR)**

(74) Representative: **Plasseraud IP**
**104 Rue de Richelieu**
**CS92104**
**75080 Paris Cedex 02 (FR)**

# (54) COPOLYMER, ANTIMICROBIAL DEODORANT COMPOSITION COMPRISING SAME, AND METHOD FOR PRODUCING SAME

(57) The present specification relates to a copolymer including a first unit derived from starch and a second unit derived from a compound represented by the following Chemical Formula 1, an antibacterial deodorant composition including the same, and a preparation method thereof.

EP 4 467 583 A1

## Description

[Technical Field]

The present specification relates to a copolymer, an antibacterial deodorant composition including the same, and a preparation method thereof.

This application claims priority to and the benefit of Korean Patent Application Nos. 10-2022-0131942 and 10-2023-0136164 filed in the Korean Intellectual Property Office on October 14, 2022 and October 12, 2023, respectively, the entire contents of which are incorporated herein by reference.

[Background Art]

In daily life, as the damage caused by microorganisms which are harmful to the human body and cause odor, such as bacteria and mold, increases, various antibacterial or deodorant materials have been developed to suppress the proliferation of or kill such microorganisms. In particular, antibacterial or deodorizing power is required for polymeric materials used in products that we commonly use. As an example, there is a need for developing an antibacterial deodorant material that is suitable for use in pet supplies (such as cat litter).

In the related art, in order to introduce antibacterial deodorant properties into polymers, a method of simply mixing antibacterial deodorant materials with polymers has been usually used. Since there is a possibility that an antibacterial deodorant material may be released, the safety of the antibacterial deodorant material used needs to be guaranteed. Therefore, in order to fundamentally solve a problem caused by the release of an antibacterial deodorant material, studies have been actively conducted on the development of an antibacterial polymer that prevents an antibacterial material from being released by copolymerizing the antibacterial deodorant material with a polymer.

[Citation List]

(Patent Document 1) Korean Patent Application Laid-Open No. 10-2009-0131847

[Detailed Description of the Invention]

[Technical Problem]

The present specification provides a copolymer, an antibacterial deodorant composition including the same, and a preparation method thereof.

[Technical Solution]

An exemplary embodiment of the present specification provides a copolymer including: a first unit derived from starch; and

a second unit derived from a compound represented by the following Chemical Formula 1.

[Chemical Formula 1]

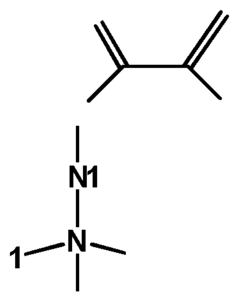

In Chemical Formula 1,

L1 is an alkylene group, any one of R1 to R3 is an alkyl group having 5 to 30 carbon atoms, and the others are the same as or different from each other, and are each independently an alkyl group having 1 to 30 carbon atoms, and

R4 is hydrogen or a methyl group.

Another exemplary embodiment of the present specification provides an antibacterial deodorant composition including the above-described copolymer.

Still another exemplary embodiment of the present specification provides a product including the antibacterial deodorant composition or prepared therefrom.

Yet another exemplary embodiment of the present specification provides a method for preparing the above-described copolymer, the method including:

reacting starch and the compound represented by the Chemical Formula 1 at one or more temperatures selected from 80°C to 140°C.

[Advantageous Effects]

The copolymer according to an exemplary embodiment of the present specification can provide improved antibacterial activity and deodorant activity.

The copolymer according to an exemplary embodiment of the present specification can solve safety problems due to the release of antibacterial materials.

The copolymer according to some exemplary embodiments of the present specification can exhibit antibacterial properties within a short period of time.

Since the copolymer according to an exemplary embodiment of the present specification has little change in antibacterial activity depending on the amount of antibacterial material used, the copolymer can exhibit antibacterial properties within an expected range even when the unevenness of concentration occurs unintentionally during application to a product. Therefore, antibacterial properties are controlled within a specific range, so that excellent antibacterial properties can be safely imparted.

The copolymer according to an exemplary embodiment of the present specification has low toxicity, and thus can solve safety problems.

[Best Mode]

Hereinafter, the present specification will be described in detail.

In the related art, in order to impart antibacterial-deodorant activity to materials, antibacterial deodorants were simply mixed with other materials, and in this case, inorganic antibacterial-deodorants or organic antibacterial-deodorants were used. The inorganic antibacterial-deodorants are expensive, easily cause discoloration of materials, and may degrade the physical properties of polymers during processing processes such as extrusion or injection. Further, the inorganic antibacterial-deodorants also have a disadvantage in that the immediate antibacterial-deodorant efficacy thereof is low. The organic antibacterial-deodorants have a disadvantage in that antibacterial-deodorant persistence deteriorates due to poor stability against the human body and poor thermal stability.

In contrast, since the copolymer according to the present specification does not include an inorganic antibacterial-deodorant, and thus overcomes disadvantages such as discoloration and deterioration in transparency, and an antibacterial-deodorant material is not included as a separate substance but is polymerized as a monomer, there is an advantage in that stability for the human body is excellent and antibacterial-deodorant persistence is maintained. In addition, when an organic antibacterial·deodorant is polymerized with other materials, the polymerization efficiency or conversion rate decreases or the characteristics of polymerized other materials are impaired in many cases, but the present specification may also overcome these disadvantages. Due to the above advantages, the copolymer of the present specification may exhibit excellent antibacterial·deodorant persistence.

That is, the copolymer of the present specification may simultaneously ameliorate the stability problem due to the release of the antibacterial·deodorant while having excellent antibacterial·deodorant activity and antibacterial·deodorant persistence.

Furthermore, the copolymer of the present specification has the advantage of being environmentally friendly by using starch, which is a natural material.

When one part "includes" one constituent element in the present specification, unless otherwise specifically described, this does not mean that another constituent element is excluded, but means that another constituent element may be further included.

In the present specification, "*" is an attachment point in the copolymer. For example, in the following Chemical Formula 2, * means both a moiety where the first units are bonded to each other and a moiety where the first unit and the second unit are bonded.

In the present specification, the "monomer" means a unit compound that may be converted into a polymer compound by a polymerization reaction, and structures derived therefrom may become a repeating unit in a polymer or

copolymer. Specifically, this means that in a state in which the corresponding compound is polymerized and bonded in the polymer, in the structure of the compound, all or a portion of two or more substituents are omitted, and a radical for being bonded to other units of the polymer is located at the position. In this case, the corresponding compound may be included in a state of being polymerized in any order and bonded in the polymer.

In the present specification, the "derived" means that a new bond is generated while a bond between at least two adjacent elements in a compound is broken, or hydrogen or a substituent is detached, and a unit derived from the compound may mean a unit which forms one or more of a main chain and a side chain in a polymer. The unit may be included in a main chain in a polymer to constitute the polymer.

In the present specification, the "weight average molecular weight" is one of the average molecular weights in which the molecular weight is not uniform and the molecular weight of any polymer material is used as a reference, and is a value obtained by averaging the molecular weight of a component molecular species of a polymer compound having a molecular weight distribution by a weight fraction.

In the present specification, among physical properties, physical properties which are affected by temperature are physical properties measured at room temperature, unless otherwise specified.

In the present specification, "room temperature" means the natural temperature at which a system has not been heated or cooled, for example, any one temperature within a range of about 10°C to 30°C, for example, a temperature of about 15°C, about 18°C, about 20°C, about 23°C, or about 25°C. Further, in the present specification, the unit of temperature is °C, unless otherwise specified.

In the present specification, when pressure among physical properties affects the results, the corresponding physical property is a physical property measured at normal pressure, unless otherwise specified.

In the present specification, "normal pressure" is the natural pressure under which a system has not been pressurized or depressurized, and refers to a pressure which is typically about 1 atmosphere (about 700 mmHg to 800 mmHg) .

In the present specification, when humidity among physical properties affects the results, the corresponding physical property is a physical property measured at a humidity that is not particularly controlled in the room temperature and normal pressure state, unless otherwise specified.

In the present specification, an "alkyl group" may be straight-chained or branched, and the number of carbon atoms thereof is not particularly limited, but is preferably 1 to 60. In an exemplary embodiment, the number of carbon atoms of the alkyl group is 1 to 30. Specific examples of the alkyl group include a methyl group, an ethyl group, a propyl group, an isopropyl group, a butyl group, an isobutyl group, a tert-butyl group, a pentyl group, a hexyl group, a heptyl group, an octyl group, a nonyl group, a decyl group, an undecyl group, a dodecyl group, a tridecyl group, and the like, but are not limited thereto.

In the present specification, an "alkylene group" means a group having two bonding positions in an alkyl group, that is, a divalent group. The above-described description on the alkyl group may be applied to the alkylene group, except for a divalent alkylene group.

In the present specification, "starch" means starch as used in the art. Specifically, starch is a polysaccharide in which many glucose units are linked by glycoside bonds, and has the following structure.

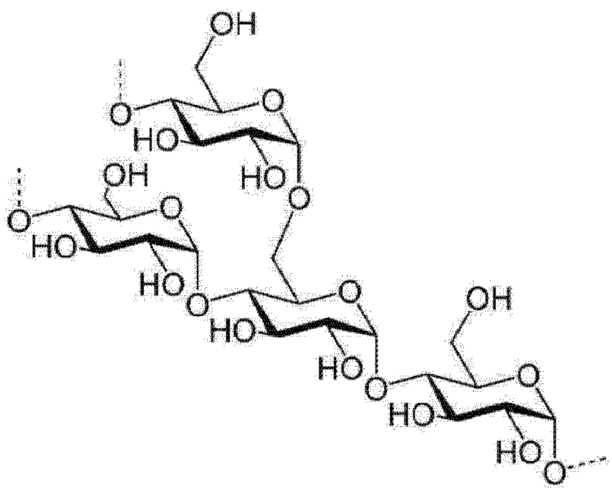

In the structure, - - - - - - means a part that is bonded to other units in starch.

In an exemplary embodiment of the present specification, the starch has a molecular weight of $10^5$ g/mol to $10^9$ g/mol, or $10^6$ g/mol to $10^8$ g/mol.

In an exemplary embodiment of the present specification, the first unit derived from the starch includes a unit represented by the following Chemical Formula 2.

[Chemical Formula 2]

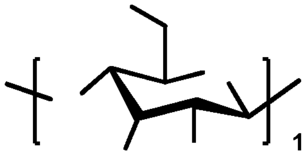

In Chemical Formula 2,

R10 to R12 are the same as or different from each other, and are each independently -OH; or -O-*, and at least one of R10 to R12 is -O-*,
n1 is an integer from 1 to $10^7$, and
* is an attachment point in the copolymer.

Specifically, the unit represented by Chemical Formula 2 means that a part of the first unit derived from the starch is grafted, or may mean that all of the first unit is grafted, in some cases.
In an exemplary embodiment of the present specification, Chemical Formula 2 is represented by any one of the following structures.

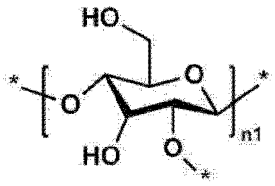
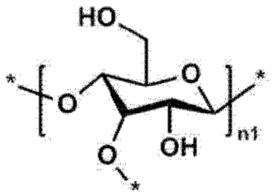
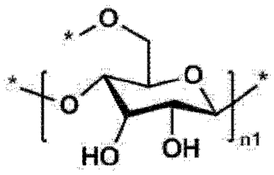

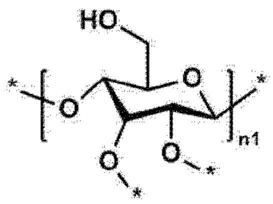
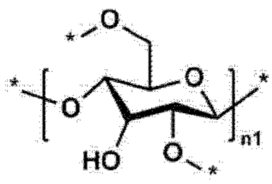
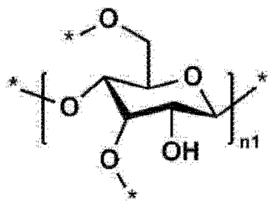

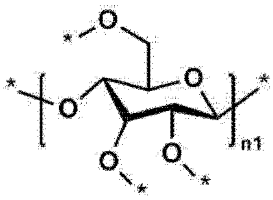

In the structures, * is an attachment point in the copolymer, and n1 is an integer from 1 to $10^7$.
Specifically, in the first unit, * is a moiety where the first units are attached to each other, or a moiety where the first unit and the second unit are attached.
In an exemplary embodiment of the present specification, n1 is an integer from 100 to $10^7$, an integer from 500 to $10^7$, an integer from $10^3$ to $10^7$, an integer from $10^4$ to $10^7$, an integer from $10^5$ to $10^7$, or an integer from $10^5$ to $10^6$.
In an exemplary embodiment of the present specification, the compound represented by Chemical Formula 1 is an antibacterial·deodorant material.
In an exemplary embodiment of the present specification, the second unit is represented by the following Chemical Formula 3.

[Chemical Formula 3]

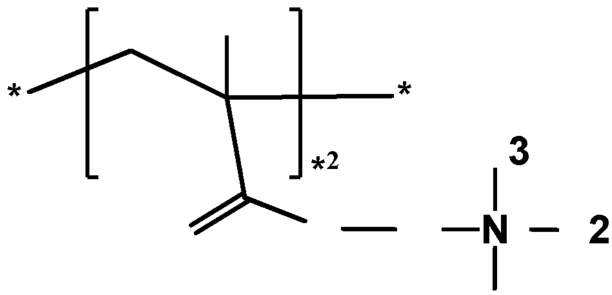

In Chemical Formula 3,

L1 is an alkylene group,
any one of R1 to R3 is an alkyl group having 5 to 30 carbon atoms, and the others are the same as or different from each other, and are each independently an alkyl group having 1 to 30 carbon atoms,
R4 is hydrogen or a methyl group,
n2 is an integer from 1 to $10^6$, and
* is an attachment point in the copolymer.

In an exemplary embodiment of the present specification, n2 is an integer from 1 to $10^6$, an integer from 1 to $10^5$, an integer from 1 to $10^4$, or an integer from 1 to 5000.
In an exemplary embodiment of the present specification, L1 is an alkylene group having 1 to 10 carbon atoms.
In an exemplary embodiment of the present specification, L1 is an alkylene group having 1 to 5 carbon atoms.
In an exemplary embodiment of the present specification, L1 is a methylene group; an ethylene group; a propylene group; or a butylene group.
In an exemplary embodiment of the present specification, any one of R1 to R3 is an alkyl group having 5 to 30 carbon atoms, and the others are the same as or different from each other, and are each independently an alkyl group having 1 to 20 carbon atoms.
In an exemplary embodiment of the present specification, any one of R1 to R3 is an alkyl group having 5 to 30 carbon atoms, and the others are the same as or different from each other, and are each independently an alkyl group having 1 to 10 carbon atoms.
In an exemplary embodiment of the present specification, any one of R1 to R3 is an alkyl group having 5 to 20 carbon atoms, and the others are the same as or different from each other, and are each independently an alkyl group having 1 to 20 carbon atoms.
In an exemplary embodiment of the present specification, any one of R1 to R3 is an alkyl group having 5 to 20 carbon atoms, and the others are the same as or different from each other, and are each independently an alkyl group having 1 to 10 carbon atoms.
In an exemplary embodiment of the present specification, two or more of R1 to R3 are an alkyl group having 5 to 30 carbon atoms, or the difference in carbon number between an alkyl group having the largest number of carbon atoms and an alkyl group having the smallest number of carbon atoms among R1 to R3 is 4 or more.
Specifically, three of R1 to R3 are an alkyl group having 5 to 30 carbon atoms; two of R1 to R3 are an alkyl group having 5 to 30 carbon atoms, the other one is an alkyl group having 1 to 30 carbon atoms; or the difference in carbon number between an alkyl group having the largest number of carbon atoms and an alkyl group having the smallest number of carbon atoms among R1 to R3 is 4 or more. In this case, an effect in which antibacterial activity is excellent is exhibited.
In an exemplary embodiment of the present specification, a difference in the number of carbon atoms between the alkyl group having the largest number of carbon atoms and the alkyl group having the smallest number of carbon atoms of 4 or more means that the asymmetry is large, and the difference in the number of carbon atoms may be 4 to 30, 5 to 30, or 5 to 10.
In an exemplary embodiment of the present specification, the compound represented by Chemical Formula 1 is any one of the following structures.

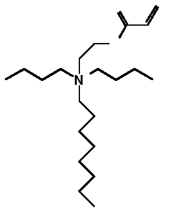
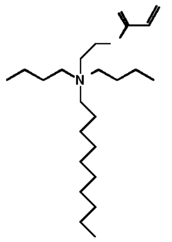
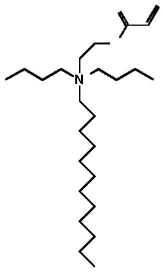
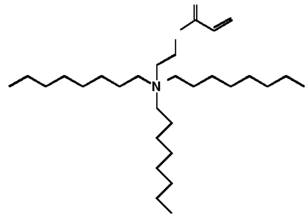

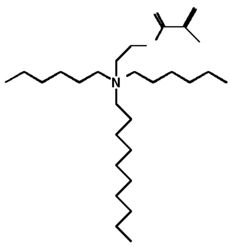
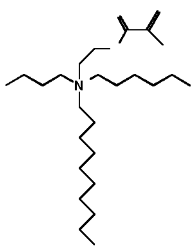
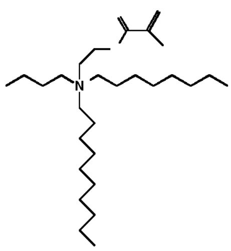
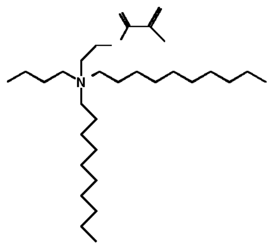

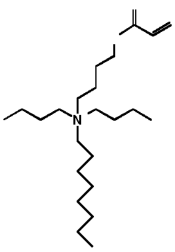
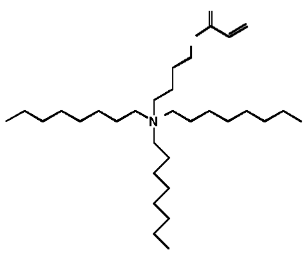
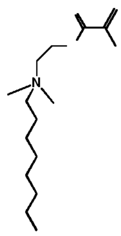
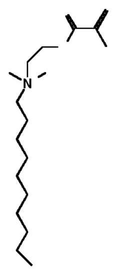
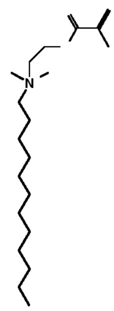

In an exemplary embodiment of the present specification, the second unit derived from the compound represented by Chemical Formula 1 is any one of the following structures.

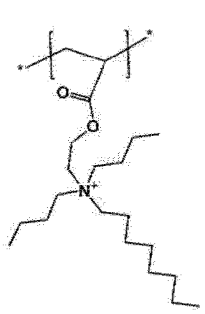
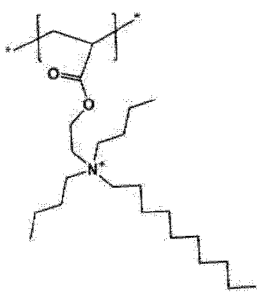
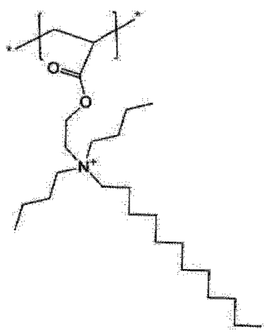

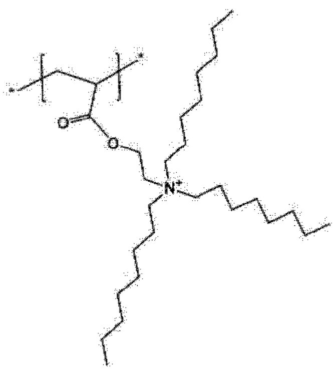
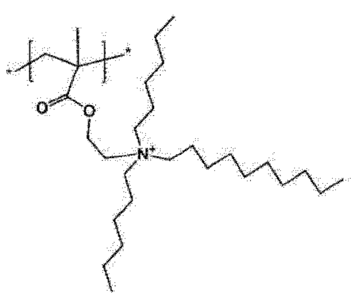

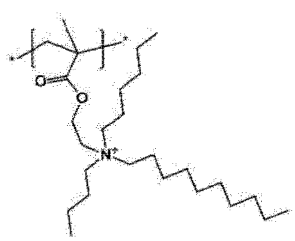
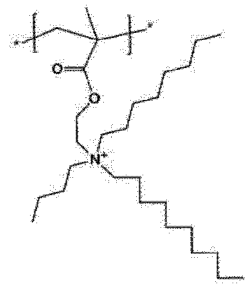
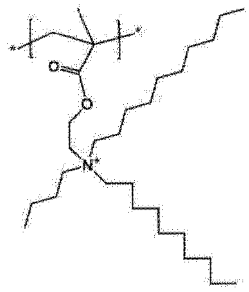

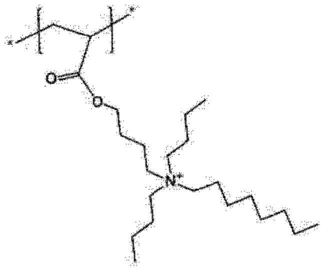
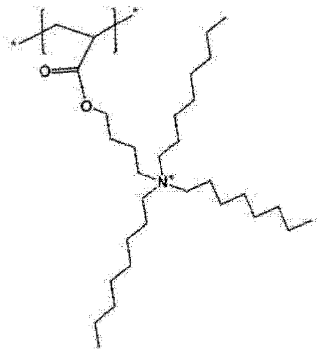

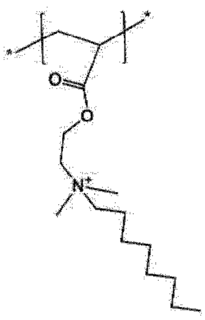
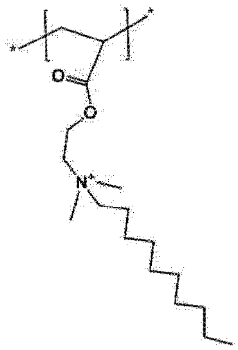
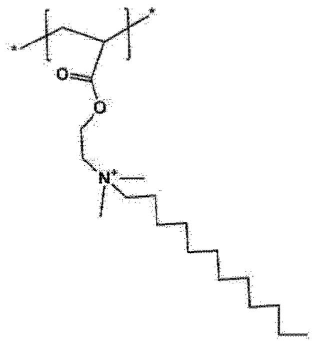

In the structures, * is an attachment point in the copolymer.

In an exemplary embodiment of the present specification, since the compound represented by Chemical Formula 1 exhibits cationic properties, the compound may be present in a form where a salt is formed along with a group exhibiting anionic properties. In this case, the group exhibiting anionic properties is not particularly limited, and materials known in the art may be used as long as the materials do not impair the purpose of the antibacterial properties. For example, the group exhibiting anionic properties may be a halogen-based anion or a sulfonate-based anion, and specifically may be Br-, but is not limited thereto.

In an exemplary embodiment of the present specification, a weight ratio of the first unit and the second unit is 100:0.5 to 100:10. Specifically, the weight ratio is 100:1 to 100:5 or 100:1 to 100:3. When the weight ratio of the first unit and the second unit satisfies the above range, there is an effect of exhibiting excellent deodorant activity, antibacterial activity, deodorant persistence, and antibacterial persistence.

The deodorant activity and antibacterial activity of the copolymer are derived from the second unit included in the copolymer. In general, cell walls of bacteria and the like are often negatively charged, and the second unit may perform a destructive action on the cell walls.

In an exemplary embodiment of the present specification, the first unit and the second unit are copolymerized. Specifically, an exemplary embodiment of the present specification provides a copolymer including: a first unit derived from starch; and a second unit derived from the compound represented by Chemical Formula 1 and copolymerized with the first unit. When the first unit and the second unit are copolymerized as described above, the antibacterial material is not included as a separate material but is polymerized as a monomer, so there is an advantage in that stability against the human body is excellent and antibacterial persistence is maintained. As an example, an exemplary embodiment of the present specification provides a copolymer in which the first unit and the second unit are grafted.

In an exemplary embodiment of the present specification, the copolymer includes a third unit represented by the

following Chemical Formula 4.

[Chemical Formula 4]

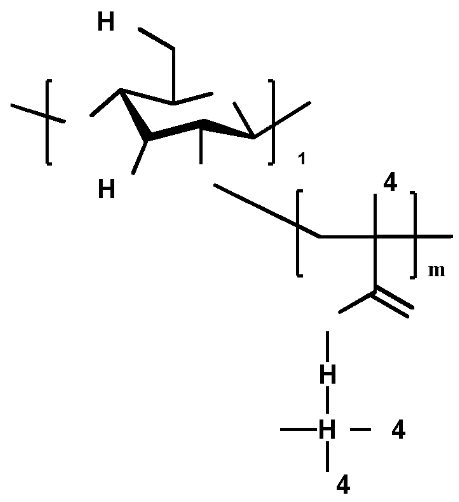

In Chemical Formula 4,

L1 is an alkylene group,
any one of R1 to R3 is an alkyl group having 5 to 30 carbon atoms, and the others are the same as or different from each other, and are each independently an alkyl group having 1 to 30 carbon atoms,
R4 is hydrogen or a methyl group,
m1 is an integer from 1 to $10^7$,
m2 is an integer from 1 to $10^6$, and
* is an attachment point in the copolymer.

In an exemplary embodiment of the present specification, a copolymer including a first unit derived from the starch; and a second unit derived from the compound represented by Chemical Formula 1 is represented by Chemical Formula 4.

According to an exemplary embodiment of the present specification, the copolymer is a random copolymer, an alternating copolymer, or a block copolymer.

In an exemplary embodiment of the present specification, the third unit represented by Chemical Formula 4 is a unit in which the first unit and the second unit are grafted.

In an exemplary embodiment of the present specification, the copolymer is composed of the third unit represented by Chemical Formula 4. Specifically, the copolymer includes a structure in which a plurality of third units are repeatedly bonded.

In an exemplary embodiment of the present specification, in the grafted copolymer, the first repeating unit is the main chain, and the second repeating unit may be grafted to the main chain in the form of side chains.

In an exemplary embodiment of the present specification, the copolymer has a weight average molecular weight (Mw) of $10^5$ g/mol to $10^9$ g/mol. Specifically, the copolymer has a weight average molecular weight of $10^6$ g/mol to $10^8$ g/mol.

In an exemplary embodiment of the present specification, the weight average molecular weight (Mw) of the copolymer may be measured using gel permeation chromatography (GPC).

In an exemplary embodiment of the present specification, the copolymer has deodorant activity.

In the present specification, having deodorant activity means that the deodorant activity measured based on the following Method 1 is 70% or more.

In an exemplary embodiment of the present specification, when the copolymer was evaluated for deodorization using the following Method 1, the deodorant activity of the copolymer against ammonia is 70% or more.

[Method 1]

2.5 mL of artificial urine inoculated with 3000±30 CFU/mL of bacteria and 1 g of the copolymer are put into a cell

culture flask, and then cultured at 35°C for 12 hours to prepare a test culture solution.

A control culture solution is prepared in the same manner as the test culture solution, except that a compressed sample of starch and glycerol is used instead of the copolymer in the method of preparing the test culture solution.

The amount of ammonia captured through the ammonia detector tube from the test culture solution and control culture solution is measured, and the deodorant activity is calculated according to the following Equation 1.

[Equation 1]

$$\text{Deodorant activity}(\%) = (1 - A_s/A_0) \times 100$$

$A_s$ = Concentration of ammonia captured in test culture solution
$A_0$ = Concentration of ammonia captured in control culture solution

In Method 1 and Equation 1 above, the test culture solution includes the copolymer according to an exemplary embodiment of the present specification, and the control culture solution does not include the copolymer.

In the present specification, the colony forming unit (CFU) means a colony forming unit, and CFU/mL means the number of CFUs per mL.

When the deodorant activity of the copolymer according to the present specification was evaluated using Method 1, only the case where the deodorant activity was 70 % or more was observed. As a result, it could be confirmed that the copolymer according to the present specification has excellent deodorant activity.

The deodorant activity may be 80% or more, 85% or more, or 90% or more in other examples. The upper limit of the deodorant activity is not particularly limited, and for example, the deodorant activity may be 100% or less, or less than 100%.

In an exemplary embodiment of the present specification, the copolymer has antibacterial properties.

In the present specification, having antibacterial properties means that the antibacterial activity measured based on the following Method 2, in other words, the bacteriostatic performance is 90% or more.

In an exemplary embodiment of the present specification, the copolymer has an antibacterial activity of 90% or more against at least one strain selected from the group consisting of Gram-positive bacteria, Gram-negative bacteria, and molds, as measured by the following Method 2.

[Method 2]

2.5 mL of artificial urine inoculated with 3000±30 CFU/mL of bacteria and 1 g of the copolymer are put into a cell culture flask, and then cultured at 35°C for 12 hours to prepare a test culture solution. Ammonia is captured from the test culture solution through an ammonia detector tube. 7.5 mL of a salt solution (0.9 wt%) is added to the test culture solution from which ammonia has been completely captured, the resulting mixture is mixed to dilute the bacterial solution, and then the diluted bacterial solution is serially diluted using a salt solution such that colony counting can be performed, and spread on a nutrient agar plate. A test sample is prepared by culturing the spread nutrient agar plate at 35°C for 18 hours.

A control sample is prepared in the same manner as the test sample, except that a compressed sample of starch and glycerol is used instead of the copolymer during the preparation of the test sample.

Antibacterial activity (%) is calculated according to the following Equation 2 by measuring the concentration of microorganisms for the test sample and the control sample.

[Equation 2]

$$\text{Antibacterial activity } (\%) = (1 - C_s/C_0) \times 100$$

$C_s$ = Concentration of microorganisms of test sample
$C_0$ = Concentration of microorganisms of control sample

In Method 2 and Equation 2 above, the test sample includes the copolymer according to an exemplary embodiment of the present specification, and the control sample does not include the copolymer.

When the antibacterial activity of the copolymer according to the present specification was evaluated using Method 2, only the case where the antibacterial activity was 90% or more was observed. As a result, it could be confirmed that the copolymer according to the present specification has excellent antibacterial activity.

The antibacterial activity may be 92% or more, 93% or more, or 94% or more in other examples. The upper limit of

the antibacterial activity is not particularly limited, and for example, the antibacterial activity may be 100% or less, or less than 100%.

As used herein, the term Gram-positive bacteria is a general term for bacteria that are stained purple when stained using the Gram staining method, and Gram-positive bacteria exhibit a purple color without discoloration even though the Gram-positive bacteria are stained with a basic dye such as crystal violet and then treated with ethanol because the cell walls of Gram-positive bacteria are composed of several layers of peptidoglycan.

In an exemplary embodiment of the present specification, the Gram-positive bacteria are selected from *Enterococcus faecalis, Staphylococcus aureus, Streptococcus pneumoniae, Enterococcus faecium* and *Lactobacillus lactis.* Specifically, the Gram-positive bacteria are any one selected from the above-described examples, but are not limited thereto.

In the present specification, the Gram-negative bacteria are a general term for bacteria that are stained red when stained with the Gram staining method, and Gram-negative bacteria have an outer membrane composed of lipid polysaccharides, lipid proteins, and/or other complex polymeric materials instead of having a cell wall with a relatively small amount of peptidoglycan compared to Gram-positive bacteria.

In an exemplary embodiment of the present specification, the Gram-negative bacteria are selected from *Proteus mirabilis, Escherichia coli, Salmonella typhi, Pseudomonas aeruginosa* and *Vibrio cholerae.* Specifically, the Gram-negative bacteria are any one selected from the above-described examples, but are not limited thereto.

In an exemplary embodiment of the present specification, the mold may be *Candida albicans,* but is not limited thereto.

In an exemplary embodiment of the present specification, the copolymer has an antibacterial activity of 90% or more against Gram-positive bacteria as measured by Method 2.

In an exemplary embodiment of the present specification, the copolymer has an antibacterial activity of 90% or more against Gram-negative bacteria as measured by Method 2.

In an exemplary embodiment of the present specification, the copolymer has an antibacterial activity of 90% or more against molds as measured by Method 2.

In an exemplary embodiment of the present specification, the copolymer has an antibacterial activity of 90% or more against Gram-positive bacteria, Gram-negative bacteria, and molds as measured by Method 2. Since the strains of the Gram-positive bacteria, Gram-negative bacteria and molds may not only induce various diseases upon contact, but also cause secondary infections, it is preferred to exhibit antibacterial properties against all of the Gram-positive bacteria, Gram-negative bacteria, and molds using one antibacterial agent.

According to an exemplary embodiment of the present specification, the bacterium used in the measurement of antibacterial activity is a Gram-negative bacterium. Specifically, the bacterium used for the evaluation of antibacterial activity is *Proteus mirabilis.*

Another exemplary embodiment of the present specification provides an antibacterial deodorant composition including the above-described copolymer.

In an exemplary embodiment of the present specification, the antibacterial deodorant composition may further include one or more of activated carbon; bentonite; a superabsorbent resin (SAP); polyethylene (PE); polypropylene (PP); polystyrene (PS); polyamide (PA); polyimide (PI); polyethylene terephthalate (PET); polyvinyl chloride (PVC); acryloyl-butadiene-styrene (ABS); and polyacrylic acid (PA).

In an exemplary embodiment of the present specification, the antibacterial deodorant composition further includes one to five, one to three, two, or one of the above-described additional components.

In an exemplary embodiment of the present specification, the antibacterial deodorant composition may be in a state where the compound and the additional component are simply mixed.

An exemplary embodiment of the present specification provides a molded article including the above-described antibacterial deodorant composition or prepared therefrom. The molded article may be specifically litter for cats, a vegetable box for a refrigerator, an automobile part, a blow molding molded article, an inflation molded article, a cast molded article, an extrusion laminate molded article, an extrusion molded article, a foam molded article, an injection molded article, a sheet, a film, a fiber, a monofilament, or a non-woven fabric, but is not limited to the examples. The automobile part may be an interior or exterior material for automobiles.

An exemplary embodiment of the present specification provides a composition for forming a copolymer for forming the above-described copolymer.

In an exemplary embodiment of the present specification, the composition for forming a copolymer includes: starch; a compound represented by Chemical Formula 1; and an initiator.

In an exemplary embodiment of the present specification, the starch is included in an amount of 55 parts by weight to 99.7 parts by weight based on 100 parts by weight of the composition for forming a copolymer. Specifically, the starch is included in an amount of 65 parts by weight to 99.5 parts by weight, or 70 parts by weight to 99.5 parts by weight. When the content of starch satisfies the above range, excellent antibacterial activity and odor control effects may be exhibited.

In an exemplary embodiment of the present specification, the compound represented by Chemical Formula 1 is

included in an amount of 0.2 parts by weight to 40 parts by weight based on 100 parts by weight of the composition for forming a copolymer. Specifically, the compound represented by Chemical Formula 1 is included in an amount of 0.5 parts by weight to 35 parts by weight, or 0.5 parts by weight to 30 parts by weight. When the content of the compound represented by Chemical Formula 1 satisfies the above range, there is an effect of exhibiting excellent antibacterial properties and antibacterial persistence.

In an exemplary embodiment of the present specification, the initiator is included in an amount of 0.1 parts by weight to 5 parts by weight based on 100 parts by weight of the composition for forming a copolymer. Specifically, the initiator is included in an amount of 0.1 parts by weight to 5 parts by weight based on 100 parts by weight of the composition for forming a copolymer. In this case, when the content of the initiator is less than 0.1 parts by weight, the polymerization reaction time is prolonged and the polymerization conversion efficiency is reduced, so that there is a concern in that productivity deteriorates. Specifically, the polymerization conversion efficiency is decreased, so that there is a disadvantage in that a large amount of residual monomer and decomposition products are generated. In contrast, when the content exceeds 5 parts by weight, the initiator is not completely consumed during the polymerization process and remains in a polymer to be finally prepared, so that there is a concern in that the physical properties of the polymer, particularly, thermal stability, and the like deteriorate.

In an exemplary embodiment of the present specification, examples of the initiator include a peroxide-based compound such as dicumyl peroxide, dipentyl peroxide, di-3,5,5-trimethylhexanoyl peroxide or dilauryl peroxide; a peroxydicarbonate-based compound such as diisopropyl peroxydicarbonate, di-sec-butyl peroxydicarbonate or di-2-ethylhexyl peroxydicarbonate; a peroxyester-based compound such as t-butylperoxy pivalate, 1,1,3,3-tetramethylbutyl peroxyneodecanoate or t-butyl peroxyneodecanoate; an azo-based compound such as azobisisobutyronitrile (AIBN) and azobis-2,4-dimethylvaleronitrile; a hydroperoxide-based compound such as t-butyl hydroperoxide; or a sulfate-based compound such as sodium persulfate (SPS), potassium persulfate or ammonium persulfate; or the like, and any one thereof or a mixture of two or more thereof may be used, but the initiator is not limited to the examples.

In an exemplary embodiment of the present specification, the composition for forming a copolymer further includes a plasticizer. The plasticizer can be used without limitation as long as the plasticizer is a material used in the art. For example, the plasticizer may be glycerol, but is not limited thereto.

In an exemplary embodiment of the present specification, the plasticizer is included in an amount of 0.5 parts by weight to 50 parts by weight based on 100 parts by weight of the composition for forming a copolymer. Specifically, the plasticizer is included in an amount of 5 parts by weight to 40 parts by weight. When the content of the plasticizer satisfies the above range, there is an effect of improving the processability of the copolymer.

In an exemplary embodiment of the present specification, the composition for forming a copolymer further includes a solvent. The solvent may be included in an amount other than the above-described materials based on 100 parts by weight of the composition for forming a copolymer.

In an exemplary embodiment of the present specification, the type of solvent mentioned above is not limited, but for example, water, methanol, ethyl alcohol, isopropyl alcohol, and the like may be used.

An exemplary embodiment of the present specification provides a method for preparing the above-described copolymer, the method including: reacting starch and the monomer represented by Chemical Formula 1 at one or more temperatures selected from 80°C to 140°C (a) as a method for preparing the above-described copolymer.

In an exemplary embodiment of the present specification, the specific description on the starch mentioned in the preparation method and the monomer represented by Chemical Formula 1 is the same as that described on the copolymer.

In an exemplary embodiment of the present specification, the method may be performed under temperature conditions in which one or more temperatures selected from the above-mentioned 80°C to 140°C are varied within a temperature range of 80°C to 140°C, and may be performed at any one temperature selected from the above-described temperature range. For example, the method may be performed at 80°C, 85°C, 90°C, 95°C, 100°C, 105°C, 110°C, 115°C, 120°C, 125°C, 130°C, 135°C, or 140°C. Furthermore, the method may be performed while increasing the temperature from 80°C to 140°C.

In an exemplary embodiment of the present specification, the reaction time in the reacting of the starch and the monomer may vary depending on the reaction temperature and reactant. For example, when the reaction is performed at 80°C, the reaction time may be 10 minutes to 24 hours. Specifically, the reaction time may be 10 minutes to 16 hours, 10 minutes to 10 hours, 10 minutes to 5 hours, 10 minutes to 3 hours, or 10 minutes to 1 hour. Further, when the reaction temperature or reactant is different from that in the Preparation Examples to be described below, the reaction time may vary.

In an exemplary embodiment of the present specification, the reacting of the starch and the monomer may include: preparing a mixture of the starch and the monomer represented by Chemical Formula 1 (a1); and reacting the mixture (a2).

In an exemplary embodiment of the present specification, in the reacting of the mixture (a2), the reaction time is the same as that described above.

In an exemplary embodiment of the present specification, the preparing of the mixture (a1) may include:

preparing starch and a monomer represented by Chemical Formula 1 (a1-1); preparing Mixture A by mixing the starch with a solvent (a1-2); and preparing Mixture B by mixing Mixture A with the monomer represented by Chemical Formula 1 (a1-3).

In an exemplary embodiment of the present specification, the preparing of Mixture B (a1-3) may include a process of mixing Mixture A with the monomer represented by Chemical Formula 1 while applying pressure. The mixing method while applying pressure can be used without limitation as long as it is a method used in the art, and for example, an inter mixer may be used.

In an exemplary embodiment of the present specification, the preparing of Mixture B (a1-3) may be performed at 10 rpm to 500 rpm for 5 minutes to 5 hours.

In an exemplary embodiment of the present specification, the method for preparing a copolymer may further include washing the reaction product (b). Specifically, the reaction product may be washed using one or more of an organic solvent such as water, ethanol, and acetone.

In an exemplary embodiment of the present specification, the method for preparing a copolymer may further include drying the reaction product (c). Specifically, the reaction product may be dried at 25°C to 140°C for 1 hour to 48 hours.

In an exemplary embodiment of the present specification, the method for preparing a copolymer may further include pulverizing the reaction product (d). The pulverization may be performed using a method used in the art, and the reaction product may be obtained in the form of sand through the pulverizing of the mixture.

In an exemplary embodiment of the present specification, the method for preparing a copolymer may further include: one or more of washing the reaction product (b); drying the reaction product (c); and pulverizing the reaction product (d).

In an exemplary embodiment of the present specification, the method for preparing a copolymer may include reacting starch and a monomer represented by Chemical Formula 1 (a); and one or more of washing the reaction product (b), drying the reaction product (c), and pulverizing the reaction product (d).

[Mode for Invention]

Hereinafter, the present specification will be described in detail with reference to Examples for specifically describing the present specification. However, the Examples according to the present specification may be modified in various forms, and it is not interpreted that the scope of the present specification is limited to the Examples described below. The Examples of the present specification are provided to more completely explain the present specification to a person with ordinary skill in the art.

**<Preparation Example 1> Preparation of monomer represented by Chemical Formula 1**

**Preparation Example 1-1. Synthesis of Compound 1**

Step 1.

(1) 0.1 mol of 2-(dibutylamino)ethanol (DBAE), 0.1 mol of trimethylamine and 0.001 mol of hydroquinone were added to 100 mL of THF (solvent).
(2) 0.1 mol of acryloyl chloride was added dropwise onto a reaction solution while stirring the materials (room temperature).
(3) The resulting mixture was stirred for 2 hours.
(4) After a triethylamine salt was removed by filtering the mixture, the solvent was removed by a rotary evaporator.
(5) The residue was dried under vacuum at 83°C to 87°C.

Step 2.

(1) The product of Step 1 and 1-bromooctane were dissolved at 50 wt% in acrylonitrile (solvent) at a molar ratio of 1:1.
(2) Subsequently, p-methoxyphenol, which is a polymerization inhibitor, was added (ratio with reaction product is 1:0.001 (eq)).
(3) The resulting mixture was reacted at 50°C for 20 hours.
(4) After static precipitation (MTBE:reaction solution = 15:1 (volume ratio)) in methyl t-butyl ether (MTBE), the mixture

was filtered.
(5) The filtered mixture was vacuum dried at 45°C to prepare Compound 1.

**Preparation Example 1-2. Synthesis of Compounds 2 and 3**

Compounds 2 and 3 were prepared in the same manner as in Preparation Example 1-1, except that 1-bromodecane (preparation of Compound 2) or 1-bromododecane (preparation of Compound 3) was used instead of 1-bromooctane in (1) of Step 2 of Preparation Example 1-1.

**Preparation Example 1-3. Synthesis of Compound 4**

Compound 4 was prepared in the same manner as in Preparation Example 1-1, except that 2-(dioctylamino) ethanol (DOAE) was used instead of 2-(dibutylamino)ethanol in (1) of Step 1 of Preparation Example 1-1.

**Preparation Example 1-4. Synthesis of Compound 5**

Step 1.

(1) 0.1 mol of 2-(dihexylamino) ethanol (DHAE), 0.1 mol of trimethylamine and 0.001 mol of hydroquinone were put into 100 mL of THF (solvent).
(2) 0.1 mol of methacryloyl chloride was added dropwise onto a reaction solution while stirring the materials (room temperature).
(3) The resulting mixture was stirred for 2 hours.
(4) After a triethylamine salt was removed by filtering the mixture, the solvent was removed by a rotary evaporator.
(5) The residue was dried under vacuum at 83°C to 87°C.

Step 2.

(1) The product of Step 1 and 1-bromodecane were dissolved at 50 wt% in acrylonitrile (solvent) at a molar ratio of 1:1.
(2) Subsequently, p-methoxyphenol, which is a polymerization inhibitor, was added (ratio with reactants is 1:0.001 (eq)).
(3) The resulting mixture was reacted at 50°C for 20 hours.
(4) After static precipitation (MTBE:reaction solution = 15:1 (volume ratio)) in methyl t-butyl ether (MTBE), the mixture was filtered.
(5) The filtered mixture was vacuum dried at 45°C to prepare Compound 5.

**Preparation Example 1-5. Synthesis of Compound 6**

Compound 6 was prepared in the same manner as in Preparation Example 1-4, except that 2-(butylhexylamino) ethanol (BHAE) was used instead of 2-(dihexylamino)ethanol in (1) of Step 1 of Preparation Example 1-4.

**Preparation Example 1-6. Synthesis of Compound 7**

Compound 7 was prepared in the same manner as in Preparation Example 1-4, except that 2-(butyloctylamino) ethanol (BOAE) was used instead of 2-(dihexylamino)ethanol in (1) of Step 1 of Preparation Example 1-4.

**Preparation Example 1-7. Synthesis of Compound 8**

Compound 8 was prepared in the same manner as in Preparation Example 1-4, except that 2-(butyldecylamino) ethanol (BOAE) was used instead of 2-(dihexylamino)ethanol in (1) of Step 1 of Preparation Example 1-4.

**Preparation Example 1-8. Synthesis of Compound 9**

Compound 9 was prepared in the same manner as in Preparation Example 1-1, except that 2-(dibutylamino)butanol (DBAB) was used instead of 2-(dibutylamino)ethanol in (1) of Step 1 of Preparation Example 1-1.

**Preparation Example 1-9. Synthesis of Compound 10**

Compound 10 was prepared in the same manner as in Preparation Example 1-1, except that 2-(dioctylamino) butanol (DOAB) was used instead of 2-(dibutylamino)ethanol in (1) of Step 1 of Preparation Example 1-1.

**Preparation Example 1-10. Synthesis of Compound 11**

(1) 1-bromooctane (preparation of Compound 11), 2-(dimethylamino)ethyl methacrylate, 4-methoxyphenol and acetonitrile were sequentially put into a two-neck round bottom flask (RBF).
(2) The resulting mixture was reacted at 60°C for 6 hours.
(3) After static precipitation (MTBE:reaction solution = 15:1 (volume ratio)) in methyl t-butyl ether (MTBE), the mixture was filtered.
(4) The resulting product was dried under vacuum at 45°C.

**Preparation Example 1-11. Synthesis of Compounds 12 and 13**

Compounds 12 and 13 were prepared in the same manner as in Preparation Example 1-10, except that 1-bromodecane (preparation of Compound 12) or 1-bromododecane (preparation of Compound 13) was used instead of 1-bromooctane in Preparation Example 1-10.
Although a static precipitation method of adding a reactant to a nonsolvent was used in Preparation Examples 1-1 to 1-11, a reverse precipitation method of adding a nonsolvent to a reactant may also be used. Furthermore, in addition to 15:1, other ratios of MTBE and reaction solution may be used, and for example, 12:1 and 26:1 may be used.
The structures of Compounds 1 to 13 prepared in Preparation Examples 1-1 to 1-11 above are as follows.

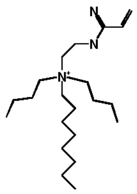
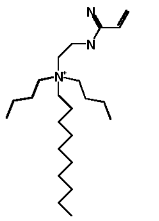
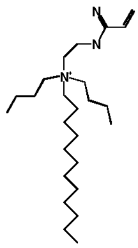
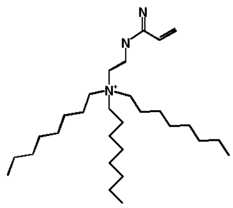

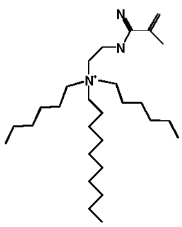
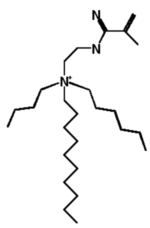
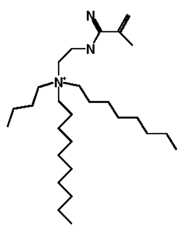
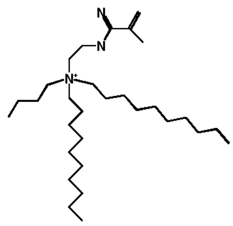

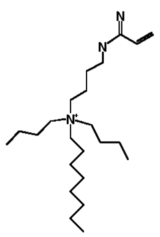
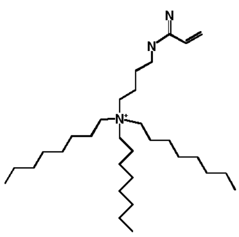
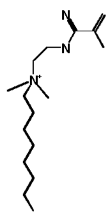
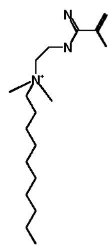
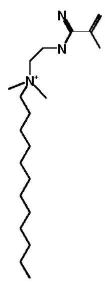

**<Preparation Example 2> Preparation of copolymer**

**Preparation Example 2-1.**

28.44 g of glycerol and 14.39 g of water were added to 100 g of corn starch (target), 2 g of Compound 12 and 1.33 g of SPS were additionally added thereto, and then the resulting mixture was uniformly mixed. The corresponding mixture was put into an internal mixer heated to 100°C and reacted at 50 rpm for 40 minutes, and then a sample in the form of dough was obtained. The corresponding sample was dried at a temperature of 80°C for 16 hours and then pulverized to obtain Sample 1 in the form of sand.

**Preparation Example 2-2 (Comparative Preparation Example).**

Sample 2 in the form of sand was obtained in the same manner as in Preparation Example 2-1, except that Compound 12 and SPS were not added.

**Preparation Example 2-3 (Comparative Preparation Example).**

28.44 g of glycerol and 14.39 g of water were added to 100 g of cellulose (Sigma-Aldrich, 40230854), 2 g of Compound 12 and 1.33 g of SPS were additionally added thereto, and then the resulting mixture was uniformly mixed. The corresponding mixture was put into an internal mixer heated to 100°C and reacted at 50 rpm for 40 minutes, but a sample was obtained in the form of a powder introduced because cellulose was not gelatinized.

**Preparation Example 2-4 (Comparative Preparation Example).**

Sample 3 in the form of sand was prepared in the same manner as in Preparation Example 2-1, except that the following Monomer A was applied instead of Compound 12 in Preparation Example 2-1.

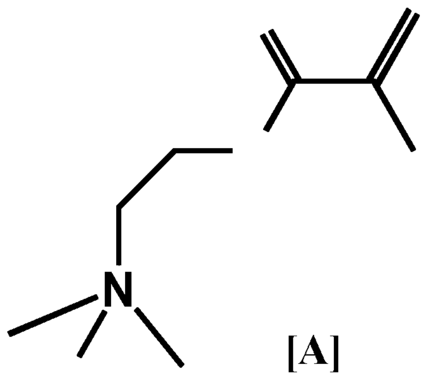

[A]

It was confirmed through HPLC measurement that Samples 1 and 3 were prepared. Specifically, 0.2 g of the target sample was added to 10 mL of salt solution, the resulting mixture was shaken at room temperature for 24 hours, and then the resulting eluate was measured by HPLC. As a result of HPLC measurement, by confirming that no residual monomer was detected, it was confirmed that the target sample had been prepared.

**<Experimental Example 1> Measurement of deodorant activity**

**Experimental Example 1-1.**

2.5 mL of artificial urine inoculated with 3000±30 CFU/mL of Proteus mirabilis bacteria and 1 g of Sample 1 prepared in Preparation Example 2-1 were put into a cell culture flask, and then cultured at 35°C for 12 hours to prepare a test culture solution.

A control culture solution was prepared in the same manner as the test culture solution, except that Sample 2 prepared in Preparation Example 2-2 above was used instead of Sample 1 in the method of preparing the test culture solution.

The amount of ammonia captured through the ammonia detector tube from the test culture solution and control culture solution was measured, and the deodorant activity was calculated according to the following Equation 1.

[Equation 1]

$$\text{Deodorant activity}(\%) = (1 - A_s/A_0) \times 100$$

$A_s$ = Concentration of ammonia captured in test culture solution
$A_0$ = Concentration of ammonia captured in control culture solution

**Comparative Example 1-1.**

The deodorant activity was calculated in the same manner as in Experimental Example 1-1, except that Sample 3 prepared in Preparation Example 2-4 was used instead of Sample 1 in Experimental Example 1-1 above.

The amount of ammonia captured and the calculated deodorant activity measured in Experimental Example 1-1 and Comparative Example 1-1 above are shown in the following Table 1.

**Comparative Example 1-2.**

2.5 mL of artificial urine inoculated with 3000±30 CFU/mL of Proteus mirabilis bacteria and 1 g of Sample 2 prepared in Preparation Example 2-2 were put into a cell culture flask, and then cultured at 35°C for 12 hours to prepare a test culture solution.

The amount of ammonia captured through an ammonia detector tube from the test culture solution was measured, and is shown in the following Table 1.

[Table 1]

| | Experimental Example 1-1 | Comparative Example 1-1 | Comparative Example 1-2 |
|---|---|---|---|
| #1 | 40 | 600 | 600 |
| #2 | 90 | 510 | More than 700 |
| Average | 65 | 555 | More than 650 |
| Deodorant activity | 90.0 | 14.6 | - |

In Table 1 above, a first measurement result and a second measurement result are described as #1 and #2, respectively, by repeatedly measuring the amount of ammonia detected (ppm) for the same sample, and the average is the average value of ammonia detected in #1 and #2.

From Table 1 above, it can be confirmed that the copolymer according to an exemplary embodiment of the present invention (Sample 1, Experimental Example 1-1) has an amount of ammonia detected, which is as low as less than 100. In contrast, it can be confirmed that both a copolymer using a compound in which all of R1 to R3 are a methyl group (Sample 3, Comparative Example 1-1) and a polymer composed only of starch (Comparative Example 1-2) have an amount of ammonia detected, which is as high as 500 or more.

Furthermore, from Table 1 above, it can be confirmed that the copolymer according to an exemplary embodiment of the present invention (Sample 1, Experimental Example 1-1) has a deodorant activity of 70 % or more, compared to a polymer composed only of starch (Sample 2, Control Culture Solution). In contrast, it can be confirmed that in the case of a polymer to which a compound in which all of R1 to R3 are a methyl group is applied (Sample 3, Comparative Example 1-1), the deodorant activity is as low as 20% or less.

Therefore, from Table 1 above, it can be confirmed that the copolymer according to an exemplary embodiment of the present invention has excellent deodorant activity.

**<Experimental Example 2> Measurement of antibacterial activity**

**Experimental Example 2-1.**

2.5 mL of artificial urine inoculated with 3000±30 CFU/mL of Proteus mirabilis bacteria and 1 g of Sample 1 prepared in Preparation Example 2-1 were put into a cell culture flask, and then cultured at 35°C for 12 hours to prepare a test culture solution. Ammonia was captured from the test culture solution through an ammonia detector tube. 7.5 mL of a salt solution (0.9 wt%) was added to the test culture solution from which ammonia had been completely captured, the resulting mixture was mixed to dilute the bacterial solution, and then the diluted bacterial solution was serially diluted using

a salt solution such that colony counting could be performed, and spread on a nutrient agar plate. A test sample was prepared by culturing the spread nutrient agar plate at 35°C for 18 hours.

A control sample was prepared in the same manner as the test sample, except that Sample 2 prepared in Preparation Example 2-2 above was used instead of Sample 1 in the method of preparing the test sample.

Antibacterial activity (%) was calculated according to the following Equation 2 by measuring the concentration of microorganisms for the test sample and the control sample.

[Equation 2]

$$\text{Antibacterial activity (\%)} = (1 - C_s/C_0) \times 100$$

$C_s$ = Concentration of microorganisms of test sample
$C_0$ = Concentration of microorganisms of control sample

**Comparative Example 2-1.**

The antibacterial activity was calculated in the same manner as in Experimental Example 2-1, except that Sample 3 prepared in Preparation Example 2-4 was used instead of Sample 1 in Experimental Example 2-1 above.

The log CFU value and antibacterial activity calculated based on the concentration of microorganisms measured in Experimental Example 2-1 and Comparative Example 2-1 above are shown in the following Table 2.

**Comparative Example 2-2.**

2.5 mL of artificial urine inoculated with 3000±30 CFU/mL of Proteus mirabilis bacteria and 1 g of Sample 2 prepared in Preparation Example 2-2 were put into a cell culture flask, and then cultured at 35°C for 12 hours to prepare a test culture solution. 7.5 mL of a salt solution (0.9 wt%) was added to the test culture solution, the resulting mixture was mixed to dilute the bacterial solution, and then the diluted bacterial solution was serially diluted using a salt solution such that colony counting could be performed, and spread on a nutrient agar plate. A test sample was prepared by culturing the spread nutrient agar plate at 35°C for 18 hours.

The concentration of microorganisms of the test sample was measured and the log CFU values were calculated, and are shown in the following Table 2.

[Table 2]

| | Experimental Example 2-1 | Comparative Example 2-1 | Comparative Example 2-2 |
|---|---|---|---|
| #1 | 6.21 | 7.27 | 7.41 |
| #2 | 5.92 | 7.08 | 7.26 |
| Average | 6.09 | 7.18 | 7.34 |
| Antibacteria I activity | 94.38 | 29.97 | - |

In Table 2 above, a log CFU value measured at the first time and a log CFU value measured at the second time are described as #1 and #2, respectively, by repeatedly measuring the CFU value for the same sample, and the average is the average log CFU value of CFU values measured in #1 and #2. From Table 2 above, it can be confirmed that the copolymer according to an exemplary embodiment of the present invention (Sample 1, Experimental Example 2-1) has an antibacterial activity of 90% or more, which is excellent. In contrast, it can be confirmed that in the case of a polymer composed only of starch (Sample 2, Comparative Example 2-2) or a copolymer using a compound in which all of R1 to R3 are a methyl group in Chemical Formula 1 (Sample 3, Comparative Example 2-1), there is no antibacterial activity or the antibacterial activity is as low as 30% or less.

**Claims**

**1.** A copolymer comprising:

a first unit derived from starch; and

a second unit derived from a compound represented by the following Chemical Formula 1:

[Chemical Formula 1]

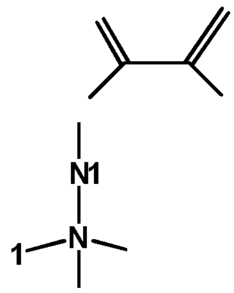

wherein, in Chemical Formula 1,
L1 is an alkylene group,
any one of R1 to R3 is an alkyl group having 5 to 30 carbon atoms, and the others are the same as or different from each other, and are each independently an alkyl group having 1 to 30 carbon atoms, and
R4 is hydrogen or a methyl group.

**2.** The copolymer of claim 1, wherein the first unit derived from the starch comprises a unit represented by the following Chemical Formula 2:

[Chemical Formula 2]

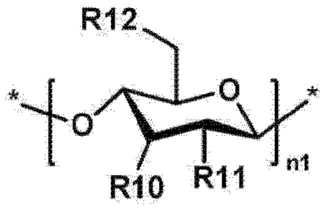

in Chemical Formula 2,

R10 to R12 are the same as or different from each other, and are each independently -OH; or -O-*, and at least one of R10 to R12 is -O-*,
n1 is an integer from 1 to $10^7$, and
* is an attachment point in the copolymer.

**3.** The copolymer of claim 1, wherein the second unit derived from the compound represented by Chemical Formula 1 is represented by the following Chemical Formula 3:

[Chemical Formula 3]

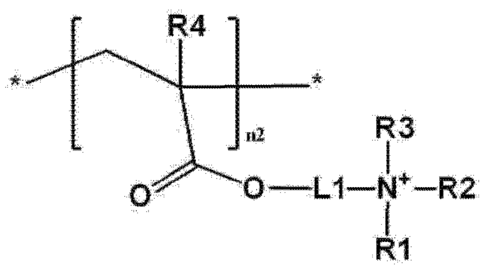

in Chemical Formula 3,

L1 is an alkylene group,
any one of R1 to R3 is an alkyl group having 5 to 30 carbon atoms, and the others are the same as or different from each other, and are each independently an alkyl group having 1 to 30 carbon atoms,
R4 is hydrogen or a methyl group,
n2 is an integer from 1 to $10^6$, and
* is an attachment point in the copolymer.

4. The copolymer of claim 1, wherein the compound represented by Chemical Formula 1 is any one of the following structures:

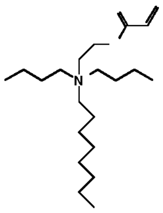
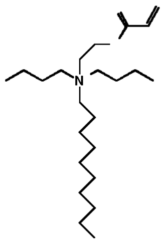
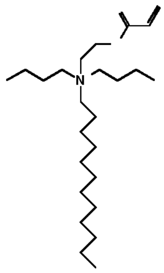
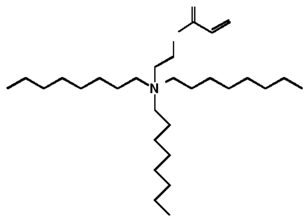
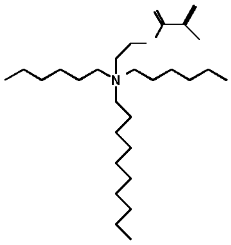
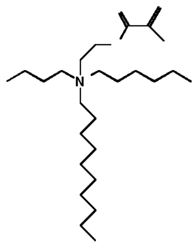
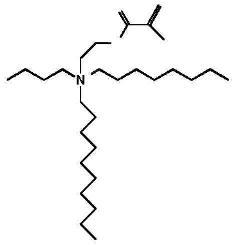
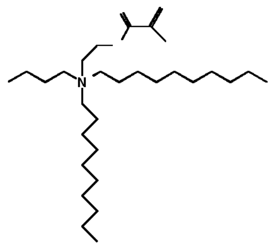
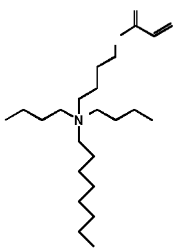
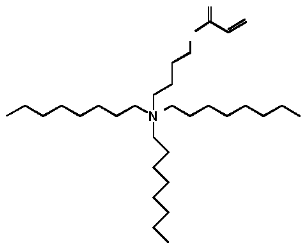
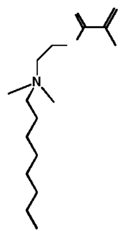
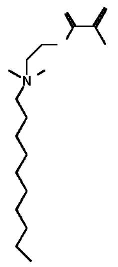
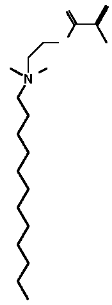
.

5. The copolymer of claim 1, wherein the copolymer comprises a third unit represented by the following Chemical Formula 4:

[Chemical Formula 4]

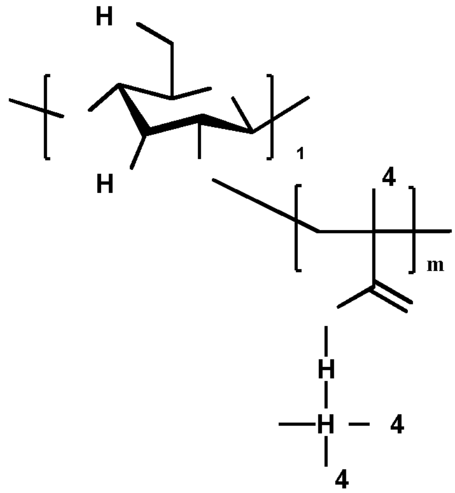

in Chemical Formula 4,

L1 is an alkylene group,
any one of R1 to R3 is an alkyl group having 5 to 30 carbon atoms, and the others are the same as or different from each other, and are each independently an alkyl group having 1 to 30 carbon atoms,
R4 is hydrogen or a methyl group,
m1 is an integer from 1 to $10^7$,
m2 is an integer from 1 to $10^6$, and
* is an attachment point in the copolymer.

**6.** The copolymer of claim 1, wherein a weight ratio of the first unit and the second unit is 100:0.5 to 100:10.

**7.** The copolymer of claim 1, wherein when the copolymer was evaluated for deodorization using the following Method 1, the deodorant activity of the copolymer against ammonia is 70% or more:
[Method 1]

2.5 mL of artificial urine inoculated with 3000±30 CFU/mL of bacteria and 1 g of the copolymer are put into a cell culture flask, and then cultured at 35°C for 12 hours to prepare a test culture solution,
a control culture solution is prepared in the same manner as the test culture solution, except that a compressed sample of starch and glycerol is used instead of the copolymer in the method of preparing the test culture solution,
the amount of ammonia captured through the ammonia detector tube from the test culture solution and control culture solution is measured, and the deodorant activity is calculated according to the following Equation 1.

[Equation 1]

$$\text{Deodorant activity} = (1 - A_s/A_0) \times 100\%$$

$A_s$ = Concentration of ammonia captured in test culture solution
$A_0$ = Concentration of ammonia captured in control culture solution

**8.** The copolymer of claim 1, wherein the copolymer has an antibacterial activity of 90% or more against at least one strain selected from the group consisting of Gram-positive bacteria, Gram-negative bacteria, and molds, as measured by the following Method 2:
[Method 2]

2.5 mL of artificial urine inoculated with 3000±30 CFU/mL of bacteria and 1 g of the copolymer are put into a cell culture flask, and then cultured at 35°C for 12 hours to prepare a test culture solution, ammonia is captured from

the test culture solution through an ammonia detector tube, 7.5 mL of a salt solution at 0.9 wt% is added to the test culture solution from which ammonia has been completely captured, the resulting mixture is mixed to dilute the bacterial solution, and then the diluted bacterial solution is serially diluted using a salt solution such that colony counting can be performed, and spread on a nutrient agar plate, a test sample is prepared by culturing the spread nutrient agar plate at 35°C for 18 hours,
a control sample is prepared in the same manner as the test sample, except that a compressed sample of starch and glycerol is used instead of the copolymer during the preparation of the test sample,
antibacterial activity in % is calculated according to the following Equation 2 by measuring the concentration of microorganisms for the test sample and the control sample.

[Equation 2]

$$\text{Antibacterial activity} = (1 - C_s/C_0) \times 100\%$$

$C_s$ = Concentration of microorganisms of test sample
$C_0$ = Concentration of microorganisms of control sample

**9.** An antibacterial deodorant composition comprising the copolymer according to any one of claims 1 to 8.

**10.** The antibacterial deodorant composition of claim 9, further comprising one or more of activated carbon; bentonite; a superabsorbent resin; polyethylene; polypropylene; polystyrene; polyamide; polyimide; polyethylene terephthalate; polyvinyl chloride; acryloyl-butadiene-styrene; and polyacrylic acid.

**11.** A product comprising the antibacterial deodorant composition according to claim 9 or prepared therefrom.

**12.** A method for preparing the copolymer according to any one of claims 1 to 8, the method comprising:

reacting starch and a compound represented by the following Chemical Formula 1 at one or more temperatures selected from 80°C to 140°C:

[Chemical Formula 1]

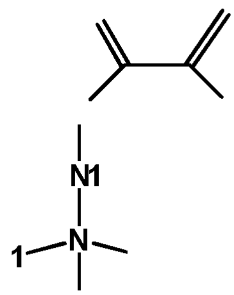

wherein, in Chemical Formula 1,
L1 is an alkylene group, any one of R1 to R3 is an alkyl group having 5 to 30 carbon atoms, and the others are the same as or different from each other, and are each independently an alkyl group having 1 to 30 carbon atoms, and R4 is hydrogen or a methyl group.

## INTERNATIONAL SEARCH REPORT

International application No.
**PCT/KR2023/015804**

**A. CLASSIFICATION OF SUBJECT MATTER**

**C08F 251/00**(2006.01)i; **C08F 220/34**(2006.01)i; **C08L 51/02**(2006.01)i; **C08L 101/00**(2006.01)i; **C08K 3/04**(2006.01)i; **C08K 3/34**(2006.01)i; **A61L 9/01**(2006.01)i; **C08B 31/00**(2006.01)i

According to International Patent Classification (IPC) or to both national classification and IPC

**B. FIELDS SEARCHED**

Minimum documentation searched (classification system followed by classification symbols)

C08F 251/00(2006.01); A01N 25/04(2006.01); A01N 33/12(2006.01); A01N 43/16(2006.01); C08B 31/00(2006.01); C08F 8/44(2006.01); C09D 7/20(2018.01); C09D 7/40(2018.01); C11D 3/48(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (Registry, Caplus), Google & keywords: 항균력(antibacterial, antibiotic, antimicrobial), 전분(starch), 4급 암모늄(quaternary ammonium), 공중합체(copolymer)

**C. DOCUMENTS CONSIDERED TO BE RELEVANT**

| Category* | Citation of document, with indication, where appropriate, of the relevant passages | Relevant to claim No. |
|---|---|---|
| Y | HUANG, M. et al. Dual functionality of a graft starch flocculant: Flocculation and antibacterial performance. Journal of Environmental Management. 2017, vol. 196, pp. 63-71.<br>See abstract; pages 64 and 68; and table 1. | 1-12 |
| Y | KR 10-2022-0046496 A (LG CHEM, LTD.) 14 April 2022 (2022-04-14)<br>See claim 1; and paragraphs [0056], [0060], [0100] and [0245]-[0269]. | 1-12 |
| A | JP 2000-154105 A (DAICEL CHEM. IND. LTD.) 06 June 2000 (2000-06-06)<br>See entire document. | 1-12 |
| A | US 2017-0360040 A1 (B.G. NEGEV TECHNOLOGIES AND APPLICATIONS LTD., AT BEN-GURION UNIVERSITY) 21 December 2017 (2017-12-21)<br>See entire document. | 1-12 |
| A | WO 2017-099668 A1 (AGENCY FOR SCIENCE, TECHNOLOGY AND RESEARCH) 15 June 2017 (2017-06-15)<br>See entire document. | 1-12 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **13 February 2024** | **13 February 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**
**Information on patent family members**

International application No.
**PCT/KR2023/015804**

| Patent document cited in search report | | | Publication date (day/month/year) | Patent family member(s) | | | Publication date (day/month/year) |
|---|---|---|---|---|---|---|---|
| KR | 10-2022-0046496 | A | 14 April 2022 | CN | 115175968 | A | 11 October 2022 |
| | | | | EP | 4086317 | A1 | 09 November 2022 |
| | | | | EP | 4086317 | A4 | 20 September 2023 |
| | | | | JP | 2023-514629 | A | 06 April 2023 |
| | | | | US | 2023-0120607 | A1 | 20 April 2023 |
| | | | | WO | 2022-075763 | A1 | 14 April 2022 |
| JP | 2000-154105 | A | 06 June 2000 | | None | | |
| US | 2017-0360040 | A1 | 21 December 2017 | EP | 3227379 | A1 | 11 October 2017 |
| | | | | EP | 3227379 | A4 | 25 July 2018 |
| | | | | EP | 3227379 | B1 | 29 July 2020 |
| | | | | IL | 252619 | B | 31 August 2020 |
| | | | | WO | 2016-088124 | A1 | 09 June 2016 |
| WO | 2017-099668 | A1 | 15 June 2017 | | None | | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 1020220131942
- KR 1020230136164
- KR 1020090131847